(19) Europäisches Patentamt
European Patent Office
Office européen des brevets

(11) EP 4 021 564 B1

(12) EUROPEAN PATENT SPECIFICATION

(45) Date of publication and mention of the grant of the patent:
**22.03.2023 Bulletin 2023/12**

(21) Application number: **20781445.0**

(22) Date of filing: **16.09.2020**

(51) International Patent Classification (IPC):
**A61N 1/36** (2006.01)     **A61B 5/00** (2006.01)
**A61B 5/24** (2021.01)

(52) Cooperative Patent Classification (CPC):
**A61N 1/36039; A61B 5/24; A61B 5/4047;**
**A61B 5/4848; A61B 5/4851; A61B 5/686;**
**A61B 5/6877; A61B 5/7221;** A61B 2505/09

(86) International application number:
**PCT/EP2020/075919**

(87) International publication number:
**WO 2021/053045 (25.03.2021 Gazette 2021/12)**

(54) **COCHLEAR IMPLANT FITTING BASED ON NEURONAL STATUS**

AUF NEURONALEM ZUSTAND BASIERENDES COCHLEA-IMPLANTAT

ADAPTATION D'UN IMPLANT COCHLÉAIRE SUR LA BASE DE L'ÉTAT NEURONAL

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **17.09.2019 EP 19197902**

(43) Date of publication of application:
**06.07.2022 Bulletin 2022/27**

(73) Proprietor: **MED-EL Elektromedizinische Geräte GmbH**
**6020 Innsbruck (AT)**

(72) Inventors:
 • **STOHL, Joshua**
   **Durham, North Carolina 27705 (US)**
 • **GARNHAM, Carolyn**
   **Hampshire S450BA (GB)**
 • **BENAV, Heval**
   **60431 Frankfurt am Main (DE)**
 • **STRAHL, Stefan**
   **37136 Ebergötzen (DE)**

(56) References cited:
 • **SCHVARTZ-LEYZAC KARA C ET AL: "Across-site patterns of electrically evoked compound action potential amplitude-growth functions in multichannel cochlear implant recipients and the effects of the interphase gap", HEARING RESEARCH, ELSEVIER SCIENCE PUBLISHERS, AMSTERDAM, NL, vol. 341, 10 August 2016 (2016-08-10), pages 50-65, XP029785935, ISSN: 0378-5955, DOI: 10.1016/J.HEARES.2016.08.002**
 • **PRADO-GUITIERREZ ET AL: "Effect of interphase gap and pulse duration on electrically evoked potentials is correlated with auditory nerve survival", HEARING RESEARCH, ELSEVIER SCIENCE PUBLISHERS, AMSTERDAM, NL, vol. 215, no. 1-2, May 2006 (2006-05), pages 47-55, XP005427388, ISSN: 0378-5955, DOI: 10.1016/J.HEARES.2006.03.006**
 • **DYAN RAMEKERS ET AL: "Auditory-Nerve Responses to Varied Inter-Phase Gap and Phase Duration of the Electric Pulse Stimulus as Predictors for Neuronal Degeneration", JOURNAL OF THE ASSOCIATION FOR RESEARCH IN OTOLARYNGOLOGY, vol. 15, no. 2, 28 January 2014 (2014-01-28), pages 187-202, XP055757405, US ISSN: 1525-3961, DOI: 10.1007/s10162-013-0440-x**

EP 4 021 564 B1

**Description**

TECHNICAL FIELD

The present invention relates to signal processing arrangements for hearing implants, and more particularly, to sound coding strategies for cochlear implants.

BACKGROUND ART

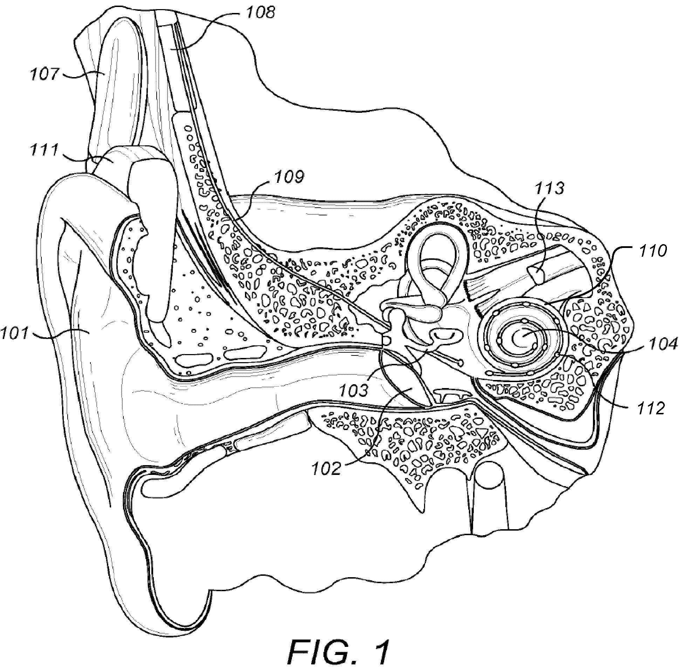

As shown in Figure 1, sounds are transmitted by a human ear from the outer ear **101** to the tympanic membrane (eardrum) **102,** which moves the bones of the middle ear **103** (malleus, incus, and stapes) that vibrate the oval window and round window openings of the cochlea **104.** The cochlea **104** is a long fluid-filled duct wound spirally about its axis for approximately two and a half turns. It includes an upper channel known as the scala vestibuli and a lower channel known as the scala tympani, which are connected by the cochlear duct. The cochlea **104** forms an upright spiraling cone with a center called the modiolus where the spiral ganglion cells (SGCs) of the auditory nerve **113** reside. These SGCs have axons projecting in one direction to the cochlear nucleus in the brainstem and they project in the other direction to the Organ of Corti as neural processes peripheral to the SGCs (hereinafter called peripheral processes) in the cochlea **104.** In response to received sounds transmitted by the middle ear **103,** sensory hair cells in the cochlea **104** function as transducers to convert mechanical motion and energy into electrical discharges in the auditory nerve **113.** These discharges are conveyed to the cochlear nucleus and patterns of induced neural activity in the nucleus are then conveyed to other structures in the brain for further auditory processing and ultimately perceiving the neural signals as sound.

Hearing is impaired when there are problems in the ability to transduce external sounds into meaningful action potentials along the neural substrate of the cochlea **104.** To improve impaired hearing, auditory prostheses have been developed. In some cases, hearing impairment can be addressed by a cochlear implant (CI), a brainstem-, midbrain- or cortical implant that electrically stimulates intact auditory nerve tissue with small currents delivered by multiple electrode contacts **112** distributed along an implant electrode array **110** that is inserted into the scala tympani of the patient cochlea **104.** For brain-stem, midbrain and cortical implants, the electrode array is located in the auditory brainstem, midbrain or cortex, respectively. Intact auditory nerve tissue or tissue with local neural survival in the context of this invention means tissue having neural structures, that when stimulated with an electrical stimulus, discharge and this neural signal is conveyed to other structures in the brain for further auditory processing and ultimately perceiving the neural signals as sound. Neural survival includes any states of the neural tissue, such as myelination, level of degeneration, functioning ion channels and the like.

Figure 1 shows some components of a typical cochlear implant system where an external microphone provides an audio signal input to an external signal processor **111** which implements one of various known signal processing schemes. For example, signal processing approaches that are well-known in the field of cochlear implants include continuous interleaved sampling (CIS) digital signal processing, channel specific sampling sequences (CSSS) digital signal processing, spectral peak (SPEAK) digital signal processing, fine structure processing (FSP) and compressed analog (CA) signal processing.

The processed signal is converted by the external signal processor **111** into a digital data format, such as a sequence of data frames, for transmission by an external coil 107 into a receiving stimulator processor **108.** Besides extracting the audio information, the receiver processor in the stimulator processor **108** may perform additional signal processing such as error correction, pulse formation, etc., and produces a stimulation pattern by means of a coding strategy (based on the extracted audio information) that is sent through electrode lead **109** to an implanted electrode array **110.** Typically, the electrode array **110** includes multiple physical stimulation contacts **112** on its surface that provide selective electrical stimulation of the cochlea **104.**

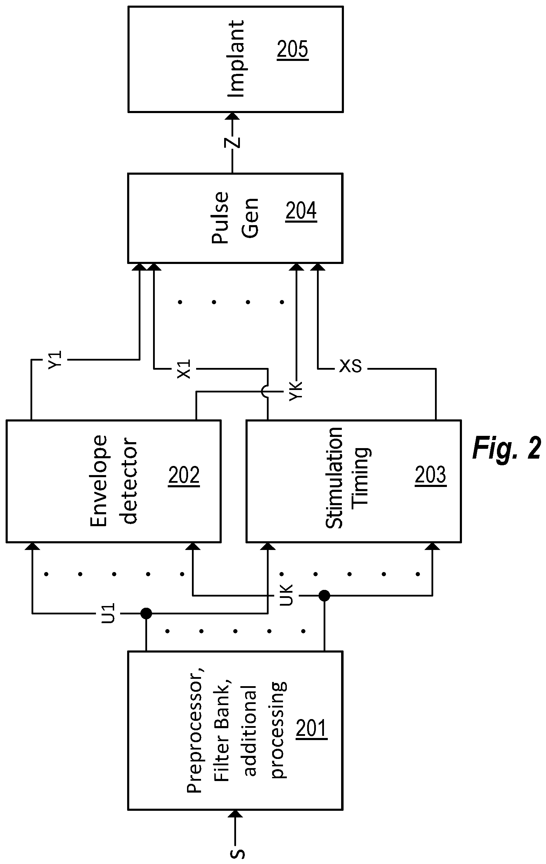

Figure 2 illustrates a typical signal processing implementation of a coding strategy. A Preprocessor Filter Bank **201** processes an audio input signal to generate $k$ band pass channel signals $U_1, ..., U_k$ that each represent a band pass channel defined by an associated band of audio frequencies. The output of the Preprocessor Filter Bank **201** goes to an Envelope Detector **202** that extracts at least a subset of $l$, where $l$ ranges from 1 to $k,$ band pass envelope signals, e.g. $Y_1, ..., Y_l$, each defined by a time varying envelope signal of the respective band pass signals $U_1, ..., U_k$. For any remaining of the subset $k-l$ envelope signals e.g. $Y_{l+1}, ..., Y_k$, Envelope Detector **202** is by-passed and the signal corresponds to the respective band pass channel signals, e.g. $U_{l+1}, ..., U_k$. The band pass signals $U_1, ...U_k$ are further input to a Stimulation Timing Module **203** that generates $s$ stimulation timing signals $X_1, ..., X_s$. The stimulation timing signals $X_1, ..., X_s$ in one example may follow a fixed time-frame or in another example reflect the temporal fine structure features of the band pass signals by detecting the negative to positive or positive to negative zero crossings of each band pass signal and in response generates a stimulation timing signal. A Pulse Generator **204** may select $n$ band pass envelope signals $Y_1, ...Y_n$ (which may include band pass signals) representing $n$ analysis channels and use the analysis channel signals $Y_1, ...Y_n$ and the stimulation timing signals $X_1, ..., X_s$ to produce for the $m$ stimulation channels the electrode

stimulation channel signals $Z_1$, ..., $Z_m$ for the electrode contacts in the implant **205**. Selection may include that a subset, none or all band pass envelope signals $Y_1$, ...,$Y_k$ are selected, i.e. $0 \le n \le k$, for one, more or all stimulation timing signals $X_1$, ..., $X_s$ and dependent on various parameters and signals, e.g. band pass envelope signals $Y_1$, ...,$Y_k$.

The Pulse Generator **204** may multiply a virtual channel matrix $W$ with the analysis channel signals $Y_1$, ...,$Y_n$ to generate stimulation channel signals $Z_1$, ..., $Z_m$ ($\vec{Z} = \overleftrightarrow{W} \times \vec{Y}$), as described in U.S. Patent US9,180,295 and US8,532,782 of the Applicant. A stimulation channel corresponds either to a physical electrode contact **112** on the electrode array **110** (physical stimulation channel) or a virtual electrode (virtual stimulation channel) created by stimulating a pair of adjacent physical electrode contacts **112** synchronously with some fixed ratio of currents. In the case of stimulating a pair of adjacent physical electrode contacts **112,** the electric fields of the pair of adj acent physical electrode contacts superimpose and the (aforementioned) ratio determines the strength of the two electric fields relative to each other and consequently the superimposed total electric field that in turn can be represented through a virtual electrode located in between the two physical electrode contacts **112**. A pair of physical electrode contacts **112** are defined to be adjacent, for as long as the superimposed total electric field can be represented through an electric field that would be generated by stimulating a single virtual electrode contact instead. For example, a ratio of 0.5 determines that the same current is applied to both physical electrode contacts **112** and in effect the total electric field approximately corresponds to one that would be generated by a virtual electrode being located approximately in the middle between the two physical electrode contacts on the electrode array **110**. A ratio of 0.3 determines that on one of the two physical electrode contacts 30% of the total delivered current is applied and 70% on the respective other physical electrode contact. In effect, this corresponds to a virtual electrode being located in between the two physical electrode contacts but (normally) closer to the contact where 70% of the current is applied.

The HiRes 120 strategy of Advanced Bionics Corporation uses active current steering and additional spectral bands. The input signal is filtered into a large number of spectral bands and fast Fourier transformation (FFT) algorithms are applied for fine spectral resolution. Hilbert processing derives temporal detail from the signals while the spectral maximum for each electrode pair is determined across all the filter bands. Stimulus location is determined from the estimated frequency of the spectral maximum. A number of spectral bands are assigned to each electrode pair and the spectral bands are delivered to locations along the electrode array by dynamically varying the proportion of current delivered simultaneously to adjacent electrodes in each electrode pair.

Another stimulation concept was described by Kasturi K. & Loizou P., Effect Of Filter Spacing On Melody Recognition: Acoustic And Electric Hearing, Journal of Acoustic Society of America, 122(2), 2007, p. EL 29-EL 34. A semitone filter bank was used to more accurately analyze melodies. A set of 12 semitone filters was assigned to an equal number of stimulation electrodes. They divided a certain frequency range into a number of channels and analyzed the melody recognition of CI users. They pointed out, that the melodies processed with the semitone filter spacing were preferred over melodies processed by the conventional logarithmic filter spacing. Stimulation through the use of virtual electrode contacts may be made even more effective if one simultaneously considers matching analysis filters or stimulation rate adjustments to the tonotopic organization of the implanted cochlea.

To collect information about the electrode - nerve interface, a commonly used objective measurement is based on the measurement of Neural Action Potentials (NAPs) such as the electrically-evoked Compound Action Potential (eCAP), as described by Gantz et al., Intraoperative Measures of Electrically Evoked Auditory Nerve Compound Action Potentials, American Journal of Otology 15 (2):137-144 (1994). In this approach the cochlear implant electrode array is usually placed at the scala tympani of the inner ear and because of this acts as recording electrode. The local response of the auditory nerve to an electrical stimulus is measured typically very close to the position of the nerve excitation. For example, the electrical stimulus is applied through one electrode contact of the electrode array and the nerve excitation is measured on the adjacent electrode contact on the electrode array. This compound neural response is caused by the super-position of synchronous potential changes across SGC membranes. The response is characterized by the amplitude between the minimum voltage (this peak is called typically N1) and the maximum voltage (peak is called typically P2). The amplitude of the eCAP at the measurement position is between 10 $\mu$V and 1800 $\mu$V. One eCAP recording paradigm is a so-called "amplitude growth function", as described by Brown et al., Electrically Evoked Whole Nerve Action Potentials In Ineraid Cochlear Implant Users: Responses To Different Stimulating Electrode Configurations And Comparison To Psychophysical Responses, Journal of Speech and Hearing Research, vol. 39:453-467 (June 1996). This function is the relation between the amplitude of the stimulation pulse and the peak-to-peak voltage of the eCAP. A State-of-the-art measurement system is for example described in Zimmerling M: Messung des elektrisch evozierten Summenaktionspotentials des Hörnervs bei Patienten mit einem Cochlea-Implantat in PhD thesis Universität Innsbruck, Institut für Angewandte Physik; 1999; and in Schoesser H, Zierhofer C, Hochmair ES. "Measuring electrically evoked compound action potentials using triphasic pulses for the reduction of the residual stimulation artefact" In: Conference on implantable auditory prostheses; 2001.

SCHVARTZ-LEYZAC KARA C ET AL: "Across-site patterns of electrically evoked compound action potential amplitude-growth functions in multichannel cochlear implant recipients and the effects of the interphase gap", HEARING RESEARCH, ELSEVIER SCIENCE PUBLISHERS, AMSTERDAM, NL, vol. 341, 10 August 2016 (2016-08-10), pages 50-65, XP029785935, ISSN: 0378-5955, DOI: 10.1016/J.HEARES.2016.08.002 discloses an experimental setup that is configured to measure the amplitude growth function for a selected channel in response to an applied electrical stimulation pulse for the selected channel, where the applied electrical pulses comprises two phases separated in time by a inter-phase-gap. The slope of the amplitude growth function is determined for various inter phase-gaps for the selected channel. This prior art discloses that there are variations in the amplitude growth function slope when the inter-phase gap is varied.

PRADO-GUITIERREZ ET AL: "Effect of interphase gap and pulse duration on electrically evoked potentials is correlated with auditory nerve survival", HEARING RESEARCH, ELSEVIER SCIENCE PUBLISHERS, AMSTERDAM, NL, vol. 215, no. 1-2, 1 May 2006 (2006-05-01), pages 47-55, XP005427388, ISSN: 0378-5955, DOI: 10.1016/J.HEARES.2006.03.006 generally disclose that variations in the inter-phase gap influence the morphology of the amplitude growth function, and amongst other things also the slope.

DYAN RAMEKERS ET AL: "Auditory-Nerve Responses to Varied Inter-Phase Gap and Phase Duration of the Electric Pulse Stimulus as Predictors for Neuronal Degeneration", JOURNAL OF THE ASSOCIATION FOR RESEARCH IN OTOLARYNGOLOGY, vol. 15, no. 2, 28 January 2014 (2014-01-28), pages 187-202, XP055757405, ISSN: 1525-3961, DOI: 10.1007/s10162-013-0440-x generally disclose that variations in the inter-phase gap influence the morphology of the amplitude growth function, and amongst other things also the slope.

In U.S. Patent Publication 20080021551 a system for optimizing pitch perception in CIs is described where a reference signal is generated and applied to an appropriate electrode on the electrode array. A probe signal having a fixed interval relationship with the reference signal is applied to an appropriate electrode on the electrode array. The location of the probe signal or reference signal is shifted until the two signals match. A frequency map is applied that uses the location at which the probe signal and the location at which the reference signal is applied when the two signals match. The frequency map is used to apply stimulus signals to correct locations within a cochlea as a function of pitch using virtual electrodes.

The prior-art disclose applying a virtual channel matrix for best tonotopic matching of the stimulation location by fine-grained frequency analysis of the audio signal and none of the above references describe a system and process for obtaining a virtual channel matrix based on local neuronal survival and without the need for fine-grained frequency analysis. Therefore, a need exists in the art to obtain a virtual channel matrix reflecting best local neural survival for systems and irrespective of the granularity for frequency analysis applied.

SUMMARY OF THE INVENTION

The present invention provides a method for developing a virtual channel matrix for mapping analysis channels to stimulation channels for a cochlear implant patient as defined in claim 1. The present invention also provides a system according claim 7 and a computer program product according to claim 15. Dependent claims define preferred embodiments of the present invention. The present invention as defined in claim 1 is directed to a method for developing a virtual channel matrix for mapping analysis channels to stimulation channels for a cochlear implant patient by selecting a stimulation channel and proceeding to measuring the amplitude growth function for the selected stimulation channel in response to commands to the cochlear implant to apply electrical stimulation pulses for the stimulation channel, where each stimulation pulse comprises a negative and positive phase separated in time by a first inter-phase-gap, and measuring the amplitude growth function for the selected stimulation channel in response to commands to the cochlear implant to apply electrical stimulation pulses for the stimulation channel, where each stimulation pulse comprises a negative and positive phase separated in time by a second inter-phase-gap, and whereby the first and second inter-phase-gaps are different. The next step in this process is determining the slopes of the measured amplitude growth functions for the stimulation channel measured with the first and second inter-phase-gaps, and calculating an indicator based at least in part on the difference of the slopes of the amplitude growth functions indicative of the local neural survival for that stimulation channel. Repeating this process for each stimulation channel where an indicator shall be derived. The final step in this process is selecting for the virtual channel matrix the stimulation channels with best local neural survival by optimizing a function based at least in part on the calculated indicators of the stimulation channels.

In some embodiments, first and second inter-phase-gaps are at least 2 microseconds. The difference between the first and second inter-phase-gaps is at least 8 microseconds, more specifically the difference of the first and second inter-phase-gaps is at least 7.9 microseconds and the inter-phase-gaps are at least 2.1 microseconds. The ratio between first and second inter-phase-gaps may be at least 4.

Embodiments of the present invention also include that the function further includes at least in part a component supporting uniform distribution of selected stimulation channels over the frequency range covered by the stimulation channels where an indicator shall be derived. Optimizing the function may include selecting the stimulation channels where the calculated indicator exceeds a pre-defined threshold or minimizing the variance of the calculated indicators. Optimizing the function may also include forming stimulation channel groups by grouping a pre-defined number of adjacent stimulation channels and selecting the stimulation channel with the largest calculated indicator in this group, reflecting the highest estimated local neuronal survival, or selection of one stimulation channel per group such that the variance of the calculated indicators from the selected stimulation channels for all groups is minimized.

The present invention according claim 7 is further directed to a system for fitting a virtual channel matrix for mapping analysis channels to stimulation channels of an implantable hearing system with a cochlear implant for electrical stimulation of the cochlea, the system comprising at least one hardware implemented, and programmable processor. The processor adapted to perform the steps of: selecting a stimulation channel and measuring the amplitude growth function for the selected stimulation channel in response to commanding the cochlear implant to apply electrical stimulation pulses for the stimulation channel, where each stimulation pulse comprises a negative and positive phase separated in time by a first inter-phase-gap. Measuring the amplitude growth function for the stimulation channel in response to commanding the cochlear implant to apply electrical stimulation pulses for the stimulation channel, where each stimulation pulse comprises a negative and positive phase separated in time by a second inter-phase-gap and whereby the first and second inter-phase-gaps are different. Determining the slopes of the measured amplitude growth functions for the stimulation channel measured with the first and second inter-phase-gaps and calculating an indicator based at least in part on the difference of the slopes of the amplitude growth functions indicative of the local neural survival for that stimulation channel. Repeating this process for each stimulation channel where an indicator shall be derived. Selecting for the virtual channel matrix the stimulation channels with best local neural survival by optimizing a function based at least in part on the calculated indicators of the stimulation channels.

Embodiments may include that the processor may be further adapted to allow setting the first and second inter-phase-gaps. The processor may restrict setting the first and second inter-phase-gaps shorter than 2 microseconds and/or the ratio between first and second inter-phase-gaps not less than 4. The processor may be adapted to store default inter-phase-gaps. Default inter-phase-gaps for the first and second inter phase gaps may include 2.1 microseconds and 30 or 7.9 microseconds.

Embodiments may include that the processor may be further adapted to apply an optimizing function that includes selecting the stimulation channels where the calculated indicator exceeds a pre-defined threshold, or selecting the stimulation channels that minimize the variance of the calculated indicators. The processor may be adapted to apply an optimizing function that is further based at least in part on the uniform distribution of selected stimulation channels over the frequency range covered by the stimulation channels where an indicator shall be derived. The processor may be further adapted to apply an optimizing function that includes forming stimulation channel groups by grouping a pre-defined number of adjacent stimulation channels and selecting the stimulation channel with the largest calculated indicator in this group reflecting the highest estimated local neuronal survival or by selection of one stimulation channel per group such that the variance of the calculated indicators from the selected stimulation channels for all groups is minimized. The present invention according claim 15 includes a computer program product for fitting a hearing device of a patient, the computer program product comprising a computer usable medium having computer readable program code thereon, the computer readable program code comprising instructions for carrying out any of the method steps individually or combined as described before.

BRIEF DESCRIPTION OF THE DRAWINGS

Figure 1 shows anatomical structures of a human ear and some components of a typical cochlear implant system.

Figure 2 shows various functional blocks in a signal processing arrangement for a cochlear implant according to a prior art arrangement.

Figure 3 shows details of stimulation pulses for stimulation channels utilizing virtual channels.

Figure 4 shows a stimulation pulse comprising a negative and positive phase separated in time by an inter-phase-gap and the thereafter measurable ECAP amplitude.

Figure 5 shows the estimated slopes of exemplary measured amplitude growth functions with various inter-phase-gap stimuli and the difference of the slopes, indicative of the local neuronal survival.

Figure 6 shows an example selection of stimulation channels based on optimized local neuronal survival.

DETAILED DESCRIPTION

Various advantages of the present invention are directed to a new coding and fitting algorithm suited for electrical stimulation specifically directed to relatively more intact neural structures nearby the electrode array. In addition to this site optimization, the present invention is advantageous to a new fitting and coding algorithm suited for the preservation or improvement of tonotopic optimized perception of the electrical stimulation.

Figure 1 shows anatomical structures of a human ear and some components of a typical cochlear implant system as known in the art. Figure 2 shows various functional blocks in a signal processing arrangement for a cochlear implant according to a prior art arrangement where the present invention finds advantageously application. The details of such an arrangement and its functioning have been set forth in the above discussion and more details are described below to the extent necessary to fully understand the application of the present invention in known or modified cochlear implant systems. In particular, the elements of Pulse Generator **204** are more fully described. It is readily understood by the skilled in the art, that the invention can be used in other implementations of virtual channels in cochlear implants systems, and the following description is understood as a mere example without limiting the invention and its application as claimed in any way.

Pulse Generator **204** weights each analysis channel signal $Y_1$ to $Y_k$ with a virtual channel matrix $W$ of stimulation amplitude weights that reflect local neural survival to produce a set of electrode stimulation channel signals $Z_1$ to $Z_m$ for providing an electric stimulation signal to the optimal location in the cochlea to nearby intact neural tissue. Matrix weighting of the stimulation pulses is described further in U.S. Patents US9,180,295 and US8,532,782. Equation 1 shows atypical virtual channel matrix $W$ of size $m \times k$:

$$W = \begin{pmatrix} W_{11} & W_{12} & \dots & W_{1k} \\ W_{21} & W_{22} & \dots & W_{2k} \\ \dots & \dots & \dots & \dots \\ W_{m1} & W_{m2} & \dots & W_{mk} \end{pmatrix} = \begin{pmatrix} 1 & 0 & 0 & \dots & 0 \\ 0 & 0.25 & 0 & \dots & 0 \\ 0 & 0.75 & 1 & \dots & 0 \\ \dots & \dots & \dots & \dots & \dots \\ 0 & 0 & 0 & 1 & \alpha \\ 0 & 0 & 0 & 0 & 1-\alpha \end{pmatrix} \quad \text{Equation 1}$$

where $m$ is the number of stimulation channels. The number of stimulation channels may be equal to, larger or smaller than the number of physical stimulation electrodes on the electrode array. Here $k$ is the number of analysis channels and $\alpha$ is an exemplary stimulation channel weighting factor in the range from 0 to 1 associated with analysis channel $k$. A negative weighting factor $W_{ij}$ indicates an electrical stimulation pulse with inverted polarity. Electrode stimulation signals $Z_1$ to $Z_m$ are obtained from analysis channel signals $Y_1$ to $Y_k$ by matrix multiplication $\overline{Z} = \overrightarrow{W} \times \overrightarrow{Y}$, or component wise expressed by $Z_l = \sum_{n=1}^{k} W_{ln} \cdot Y_n$. For example, for the $k^{th}$ analysis channel, analysis channel signal $Y_k$ is mapped by virtual channel matrix $W$ to the associated stimulation channel represented by stimulation channel signals $Z_1$ to $Z_m$:

$$Z = \begin{pmatrix} 1 & 0 & 0 & \dots & 0 \\ 0 & 0.25 & 0 & \dots & 0 \\ 0 & 0.75 & 1 & \dots & 0 \\ \dots & \dots & \dots & \dots & \dots \\ 0 & 0 & 0 & 1 & \alpha \\ 0 & 0 & 0 & 0 & 1-\alpha \end{pmatrix} \times \begin{pmatrix} 0 \\ 0 \\ 0 \\ \dots \\ 0 \\ Y_k \end{pmatrix} = \begin{pmatrix} 0 \\ 0 \\ 0 \\ \dots \\ \alpha \cdot Y_k \\ (1-\alpha) \cdot Y_k \end{pmatrix}.$$

In a further embodiment, the cochlear implant comprises a tonotopicity correction in Stimulation Timing **203** through stimulation rate-adjustment. In one embodiment Stimulation Timing **203** generates fixed sequences of stimulation timing signals in fixed time-frames that are subsequently adjusted. For example, for a cochlear implant system comprising 12 stimulation channels, each time-frame comprises a time sequence at fixed time-intervals $t$ of stimulation timing signals $X_1, ..., X_s$ as follows:

$$X(1) = (1 \quad 0 \quad 0 \quad 0 \quad 0 \quad 0 \quad 0 \quad 0)$$

$$X(2) = (0 \quad 1 \quad 0 \quad 0 \quad 0 \quad 0 \quad 0 \quad 0)$$

$$...$$

$$X(12) = (0 \quad 0 \quad 0 \quad 0 \quad 0 \quad 0 \quad 0 \quad 1)$$

where $X(t)$ represents the stimulation timing signals at time-instant $t$ within the time-frame and a one indicates that for the respective stimulation channel the Pulse Generator **204** generates a stimulation pulse. A zero value indicates no stimulation pulse generation for the respective stimulation channel. All stimulation channels in this example are stimulated with the same fixed rate, i.e. inversely proportional to the time-frame duration. In one exemplary embodiment, Stimulation Timing **203** may include a timer and a rate-adjustment vector per stimulation channel. The stimulation channel specific rate-adjustment vector may in part depend on the analysis channel signal. For example, the rate-adjustment may depend on the magnitude of the respective analysis channel signal and may be determined with the inventive fitting method as further described below. For each stimulation channel the timer is first initialized with a base-value defining the frame-rate and the rate-adjustment factor read from the rate-adjustment vector and subsequently started. Upon timer expiry, a respective stimulation timing $X(t)$ signal is generated and provided to Pulse Generator **204** that generates a stimulation pulse for that stimulation channel. The timer is re-initialized as before and re-started and the before described process starts again.

In one embodiment, reading the rate-adjustment factor from the rate-adjustment vector includes calculating from the analysis channel signal an index and returning the vector component with that specific index. The calculation may involve normalizing the analysis channel signal to within the range from 0 to 1. In one specific example for stimulation channel $e$, adjustment-vector $\vec{a}$ may be given through $\vec{a} = (a_0 \ a_1 \ a_2) = (0 \ 2 \ -5)$. The analysis channel signal $Y_e$ is normalized $Y_{n,e} = Y_e/maxvalue(Y_e)$ where $maxvalue(Y_e)$ is the maximal value $Y_e$ can become. In this example $Y_{n,e}$ is assumed to be 0.3. The index $i$ may be calculated as follows:

$$i = int[0.3 \cdot (length(\vec{a}) - 1)] = int[0.3 * 2] = 1$$

and the returned rate-adjustment factor is the $i$'s component of $\vec{a}$: $a_i = a_1 = 2$. The stimulation channel timer may be initialized with the calculated rate-adjustment factor added to the base-value of e.g. 1000, which yields 1002. The channel specific stimulation rate is therefore slightly reduced, because it takes longer for the timer to expire. A negative adjustment factor, e.g. as for $a_2 = -5$, would increase the channel specific stimulation rate. Similarly, the base-value can be changed by the rate-adjustment factor in various other ways and without limitation by multiplication, dividing or any other functional relationship. It may further be possible that the rate-adjustment factor includes the base-value, in which case the timer is initialized by the rate-adjustment factor only. In a further embodiment, rate-adjustment vector may include only a single scalar rate-adjustment factor that may be determined with the fitting method described further below. This simplification reduces computation complexity and has the advantage of lower power consumption as compared to the former method.

In another embodiment, Stimulation Timing **203** may continuously estimate the instantaneous dominating frequency of the audio signal for at least some analysis channels. For example, with the method as described in U.S. Patent Publication 20180311500. The base-value is determined from the such estimated dominant instantaneous analysis channel frequency by calculating the multiplicative inverse. This has the additional advantage, that not only the stimulation channel with optimal neuronal survival is stimulated, but further that the optimal tonotopicity mapping may be simultaneously achieved and adapted to the input audio signal. In a further embodiment, the base-value $b$ may be low-pass filtered, for example with a first-order, unity-gain IIR-filter:

$$b_{t+1} = (1 - \beta) \cdot b_t + \beta \cdot b_{new}.$$

Here $b_{t+1}$ is the updated base-value at time-instant $t + 1$, $b_t$ is the former base-value at time-instant $t$, $b_{new}$ is the new calculated base-value from the estimated dominant instantaneous analysis channel frequency and $\beta$ is the filter coefficient in the range from 0 to 1. The filter coefficient $\beta$ may be a function of $b_{new}$ and a pre-defined per analysis channel target base-value $b_{target}$. In one embodiment the function may be of sigmoidal form:

$$\beta = \frac{1}{1 + e^{+a \cdot |b_{new} - b_{target}|}}$$

where $a$ is a properly chosen scaling parameter. The target base-value $b_{target}$ may be determined from the respective analysis channel center frequency and may include the rate-adjustment factor. This has the advantage, that filtering is more robust against frequency estimation errors and the target base-value $b_{target}$ is prioritized.

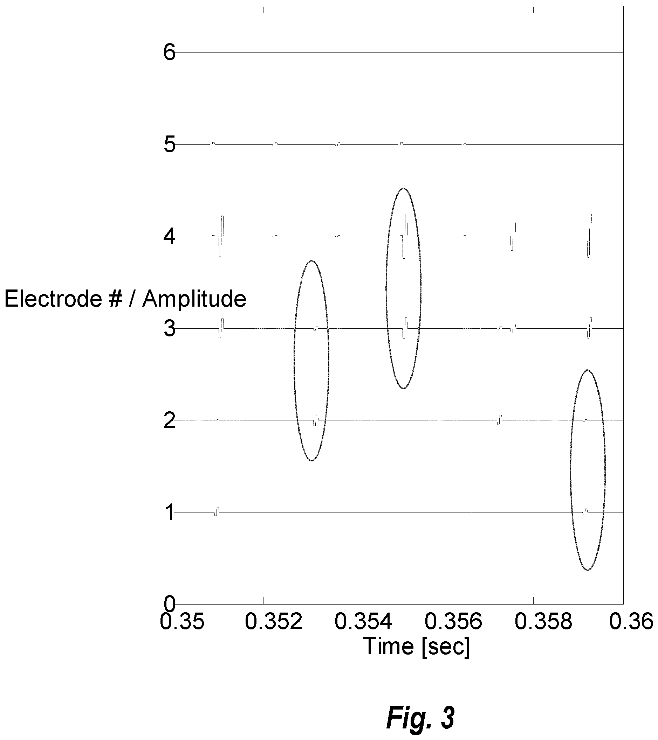

Finally, patient specific stimulation is achieved by generating mono-, bi- or triphasic monopolar pulse shapes for each electrode stimulation signal $Z_1$ to $Z_m$ to form a set of output electrode pulses $E_1$ to $E_m$ to the physical electrode contacts in the implanted electrode array through which adjacent nerve tissue is stimulated. Figure 3 shows details of simultaneous applied bi-phasic stimulation pulses at adjacent physical electrode contacts of the electrode array. Whenever one of the stimulation timing signals $X_1$ to $X_S$ indicates a stimulation event, the respective number of physical electrode contacts is activated with a set of output electrode stimulation pulses $E_1$ to $E_m$. Instead of applying a single output stimulation pulse for each selected stimulation event, frequency specific stimulation pulse sequences for one or more stimulation channels may be generated, for example channel specific sampling sequences (CSSS) as described in U.S. Patent US6,594,525 to Zierhofer. Such stimulation pulse sequences can vary in inter-pulse intervals and amplitude shape. Amplitude shapes can be based on templates, or the amplitudes can fall with a decay, e.g. with an exponential characteristic. Optionally, in addition or alternatively, the electrode stimulation signals $Z_1$ to $Z_m$ may be prior to generating pulse shapes adjusted based on a non-linear mapping that reflects patient-specific scaling from the fitting process, e.g., detection thresholds (THR) and maximum comfortable loudness (MCL) levels.

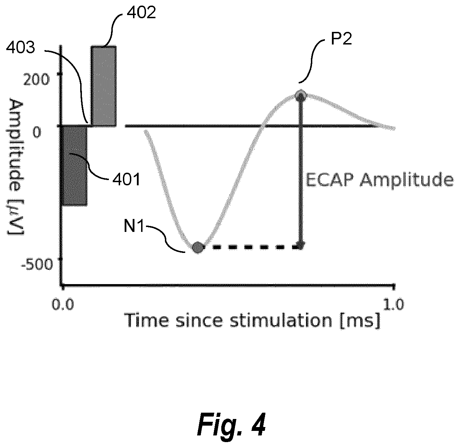

A fitting method for use in such a cochlear implant is described in the following in relation to figures 4 to 6. In a first step for developing a virtual channel matrix for mapping analysis channels to stimulation channels for a cochlear implant patient according to the present invention a stimulation channel is selected. The amplitude growth function for the selected stimulation channel is measured in response to a stimulation pulse comprising a negative and positive phase separated in time by a first inter-phase-gap. The stimulation pulse is generated by the implantable part of the cochlear implant after receiving the command and applied to the physical electrode contacts representing the stimulation channel as described before. The stimulation pulse may have the negative phase followed by the positive phase, or the positive phase followed by the negative phase. Other suitable stimulation pulse shape forms may equally be applied. Next the amplitude growth function for the stimulation channel in response to a stimulation pulse comprising a negative and positive phase separated in time by a second inter-phase-gap is measured. The first inter-phase-gap and second inter-phase-gap are different. Both slopes for the before measured amplitude growth functions are determined and an indicator based at least in part on the difference of the slopes of the amplitude growth functions, which is indicative of the local neural survival for the selected stimulation channel, is calculated. The former steps are repeated for each stimulation channel where an indicator shall be derived. Finally, for the virtual channel matrix the stimulation channels with best local neural survival are selected by optimizing a function based at least in part on the calculated indicators of the stimulation channels.

Figure 4 shows a stimulation pulse comprising a negative phase **401** and positive phase **402** separated in time by inter-phase-gap **403** as generated and delivered through the stimulation channel of the electrode array to nearby neural tissue in response to a command to the cochlear implant to apply the stimulation pulse. Thereafter the neural response is recorded through physical electrode contacts, typically adjacent to the physical electrode contacts where the stimulation pulse is applied. From the recorded signal the ECAP magnitude, which is the difference between neural response amplitudesN1 and P2, is estimated. Various known methods may be used, for example those described in the background art above. The stimulation pulse may be a single, charge balanced, rectangular biphasic pulse, i.e. positive and negative phase, where the pulse has an amplitude in a monopolar mode of stimulation. In one example, phase durations are uniform across the electrode array and equal to $30 \mu s$ per phase unless longer phase durations are required to reach MCL level on one or more electrode contacts. In this case all phase durations are increased to the minimum phase durations required to reach MCL on all electrode contacts. For determining the phase duration prior to recording ECAPs for measuring amplitude growth functions, MCLs on all electrode contacts may be measured for all physical stimulation channels using 500 $ms$ bursts of stimulation pulses with cathodic leading phase and an inter-phase-gap of 2.1 $\mu s$ at a rate of 45 stimulation pulses per second. MCLs for virtual stimulation channels may be estimated via linear interpolation from the MCLs of the immediate neighbored physical stimulation channels according the weighting factor $\alpha$ for channel $k$ as follows:

$$MCL_k = (1 - \alpha) \cdot MCL_{k-1} + \alpha \cdot MCL_{k+1}$$

This interpolation can be applied to any not measured physical stimulation channels in the same way. Measuring the MCL can be obtained in various ways, including behavioral and objective measurement techniques, for example without limitation by measurement of the stapedius reflex.

In general, measurement of the amplitude growth function includes measurement of only a part of the amplitude growth function sufficient to determine the slope. In one advantageous embodiment, the measured part of the amplitude growth function is from the largest stimulation pulse amplitude at MCL-level for that stimulation channel toward lower stimulation pulse amplitudes where a reliable an ECAP amplitude can still be measured. In addition, the pulse amplitude may be preferably in the range to capture the linear portion of the amplitude growth function, for example by adjusting the used MCL for amplitude growth measurement. The adjustment from the measured stimulation channel MCL may be dependent on the stimulation pulse inter-phase gap. The inventors have found, that the adjusted MCL for a stimulation pulse having an inter-phase gap of 30 $\mu s$ may be automatically set to a value 20 percent lower than the MCL used for a stimulation pulse having an inter-phase gap of 2.1 $\mu s$. In one embodiment, the amplitude growth function for a stimulation channel and a specific inter-phase-gap may be measured at a minimum of five uniformly spaced stimulation pulse amplitudes between 50% and 100% of the stimulation channel MCL. The slopes of the amplitude growth function may be calculated with linear regression, maximum, mean or minimal slope taken from one or more measured amplitude growth functions per stimulation channel. Various other suitable and known methods may be equally applied to calculate the slope.

The inventors also found, that the calculated indicator indicative of the local neural survival is not affected by whether the stimulation channel is a physical stimulation channel or an virtual stimulation channel. However, for calculating the indicator based at least in part on the difference of the slopes of the amplitude growth functions indicative of the local neural survival for the stimulation channel, the inter-phase gap for the stimulation pulse shall be at least 2 $\mu s$. In another embodiment, the difference between the first and second inter-phase gaps may be at least 7.9 $\mu s$. In a further embodiment, the ratio between the first and second inter-phase-gap may be at least 4. In still a further embodiment the first and second inter-phase-gap may be 2.1 $\mu s$ and 30 $\mu$, respectively.

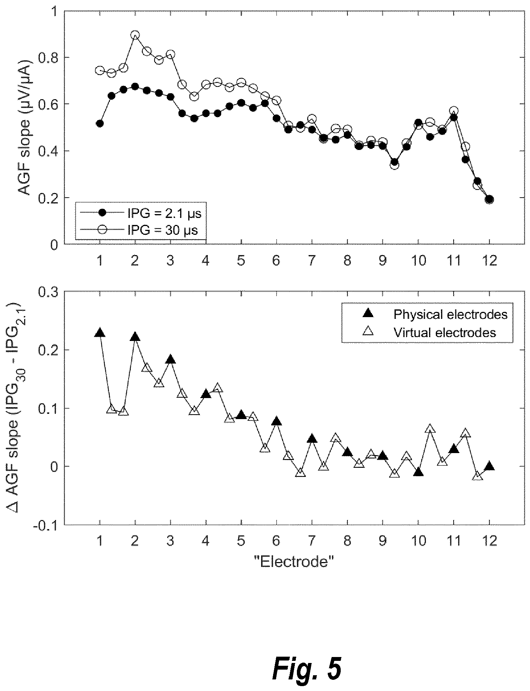

Figure 5 shows amplitude growth function slopes estimated from the measured amplitude growth functions with a stimulation pulse having an inter-phase-gap of 2.1 $\mu s$ and 30 $\mu s$ respectively over the stimulation channels in the upper diagram. The electrode array has twelve physical electrode contacts numbered from 1 to 12, each representing a physical stimulation channel. The lower diagram shows the difference of the amplitude growth function slopes, indicative of the local neuronal survival over the stimulation channels. Two virtual stimulation channels between neighbored physical stimulation channels with $\alpha$ = 0.67 and $\alpha$ = 0.33 were measured. The value of the difference of the slopes indicates local neural survival, where local means local to the stimulation channel. The higher the value of the difference, the higher the local neural survival for the stimulation channel. For example, the lower diagram shows for physical stimulation channel of physical electrode contact numbers 1 to 11 on the electrode array and virtual stimulation channels with $\alpha$ = 0.33 and $\alpha$ = 0.67. For example, the virtual channel between physical electrode contacts number 1 and 2 is represented through stimulation channel signals

$$Z = \begin{pmatrix} \alpha \cdot Y_k \\ (1 - \alpha) \cdot Y_k \\ 0 \\ ... \\ 0 \\ 0 \end{pmatrix}.$$

The slope-difference is approximately 0.4 for physical stimulation channels number 1 and 4, suggesting relatively high local neural survival for these two stimulation channels.

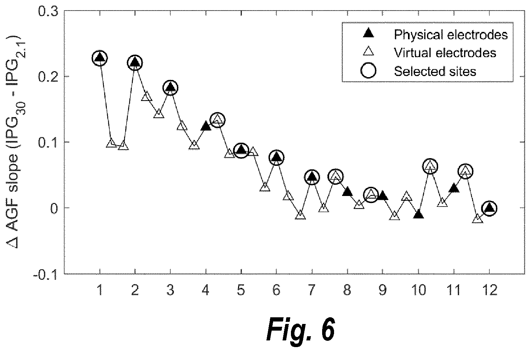

Figure 6 shows the lower diagram of Figure 5 where in addition the stimulation channels with highest estimated local neural survival are marked with a circle symbol. Physical stimulation channels are marked with solid black filled triangle symbols and named "Physical electrodes", while virtual stimulation channels are marked with a triangle symbol. For the virtual channel matrix, the stimulation channels with best local neural survival by optimizing a function based at least in part on the calculated indicators are selected. In one embodiment, optimizing the function includes selecting the stimulation channels where the calculated indicator exceeds a pre-defined threshold. Optimizing this way has the advantage that the highest total overall estimate of neural survival is selected. In another embodiment, optimizing the function includes selecting the stimulation channels that minimize the variance of the calculated indicators, this is

$$f(S) = min\big(var(S)\big)$$

with the variance var(S) and the mean value (x(S)) defined through

$$var(S) = \frac{1}{|S|} \sum_{i \in S} (x_i - \langle x(S) \rangle)^2$$

$$\langle x(S) \rangle = \frac{1}{|S|} \sum_{i \in S} x_i$$

where S is the set of indicators for selected stimulation channels comprising elements $x_i$, the calculated indicator for stimulation channel $i$. First a pre-defined number to be selected stimulation channels is determined, i.e. the cardinality of the set $S$ is determined. Typically, this number corresponds to the physical electrode contacts on the electrode array. Next, those elements for set $S$ may be selected that represent uniformly spaced stimulation channels over the electrode array. The variance $var(S)$ and mean value $\langle x(S) \rangle$ is evaluated for set $S$ and stored. Set $S$ is modified by replacing elements with the respective neighboring stimulation channel where the indicator is either closer to the mean value or higher. The variance $var(S)$ and mean value $\langle x(S) \rangle$ is evaluated for the modified set S and compared to the stored variance. If the new variance is smaller than the stored, the new calculated variance replaces the stored one. The steps are repeated until the variance is smallest.

In a further embodiment, the function further includes at least in part a component on uniform distribution of selected stimulation channels over the frequency range covered by the stimulation channels where an indicator shall be derived. In one example this part of the function may have the following form:

$$f_d(S) = \left| \frac{1}{|S|} \sum_{x_i \in S} |p(x_i) - p(x_{i+1})| - (|S| - 1) \cdot \Delta \right|$$

where $p(x_i)$ is the position of stimulation channel $i$ on the electrode array and $\Delta$ is the physical electrode spacing. The position of virtual stimulation channels is estimated to be between two physical stimulation channels proportional to weighting factor $\alpha$ for that virtual stimulation channel. In this example, optimizing the overall function may include optimizing:

$$f(S) = min\big(var(S) + f_d(S)\big)$$

In a further embodiment, optimizing the function includes forming stimulation channel groups by grouping a pre-defined number of adjacent stimulation channels and selecting the stimulation channel with the largest calculated indicator in this group, reflecting highest estimated local neuronal survival. Figure 6 shows a selection based on this embodiment. Each group is formed by a physical stimulation channel and its immediate virtual stimulation channel neighbors. Thus, the group size is three stimulation channels and the number of groups corresponds to the number of physical stimulation channels. For each group from these three stimulation channels, the one with the highest calculated indicator indicative of local neural survival is chosen. This has the advantage, that the entire electrode array is used for stimulation and regions with poor local neuronal survival remain selected and thus stimulated.

In a further embodiment, instead of selecting the stimulation channel with the highest calculated indicator indicative of local neural survival, the variance of the calculated indicators from the selected stimulation channels for all groups is minimized. This may be accomplished with the functions as described before, where modifying the set S includes building all permutations of selected channels within the groups. It is readily understood by the skilled in the art, that the inventive fitting method can be exercised in various ways. For example, the fitting method can be started by a clinician or audiologist after initiating a programming session for fitting the cochlear implant system to the patient. In other embodiments, the fitting method can be initiated by the patient itself, for example through pressing a button on the external sound processor. In another example, the inventive fitting method may be also applied in a totally implantable cochlear implant (TICI). In other embodiments, the fitting procedure may be started automatically, for example periodically or based on some measurement initiated through the cochlear implant system itself. If for example the patient has steadily increased volume on its sound processor over time, this may indicate changes in sound perception. Thus, this increase over time may be monitored and may be used to initiate the inventive fitting method. The initiation may be fully automatic or manually through the patient, clinician or audiologist after notifying the patient, clinician or audiologist in various ways of the changes and recommending initiation of re-fitting.

Embodiments of the invention may be implemented in part in any conventional computer programming language such as VHDL, SystemC, Verilog, ASM, etc. Alternative embodiments of the invention may be implemented as pre-programmed hardware elements, other related components, or as a combination of hardware and software components.

Embodiments can be implemented in part as a computer program product for use with a computer system. Such implementation may include a series of computer instructions fixed either on a tangible medium, such as a computer readable medium (*e.g.,* a diskette, CD-ROM, ROM, or fixed disk) or transmittable to a computer system, via a modem or other interface device, such as a communications adapter connected to a network over a medium. The medium may be either a tangible medium (*e.g.,* optical or analog communications lines) or a medium implemented with wireless techniques (*e.g.,* microwave, infrared or other transmission techniques). The series of computer instructions embodies all or part of the functionality previously described herein with respect to the system. Those skilled in the art should appreciate that such computer instructions can be written in a number of programming languages for use with many computer architectures or operating systems. Furthermore, such instructions may be stored in any memory device, such as semiconductor, magnetic, optical or other memory devices, and may be transmitted using any communications technology, such as optical, infrared, microwave, or other transmission technologies. It is expected that such a computer program product may be distributed as a removable medium with accompanying printed or electronic documentation (*e.g.,* shrink wrapped software), preloaded with a computer system (*e.g.,* on system ROM or fixed disk), or distributed from a server or electronic bulletin board over the network (*e.g.,* the Internet or World Wide Web). Of course, some embodiments of the invention may be implemented as a combination of both software (*e.g.,* a computer program product) and hardware. Still other embodiments of the invention are implemented as entirely hardware, or entirely software (e.g., a computer program product).

Although various exemplary embodiments of the invention have been disclosed, it should be apparent to those skilled in the art that various changes and modifications can be made which will achieve some of the advantages of the invention without departing from the invention as claimed.

## Claims

1. A method for developing a virtual channel matrix for mapping analysis channels to stimulation channels for a cochlear implant patient comprising:

    selecting a stimulation channel and measuring the amplitude growth function for the selected stimulation channel in response to commands to the cochlear implant to apply electrical stimulation pulses for the stimulation channel, where each stimulation pulse comprises a negative (401, 401a, 401b) and positive (402) phase separated in time by a first inter-phase-gap (403);
    measuring the amplitude growth function for the selected stimulation channel in response to commands to the cochlear implant to apply electrical stimulation pulses for the stimulation channel, where each stimulation pulse comprises a negative (401, 401a, 401b) and positive (402) phase separated in time by a second inter-phase-gap (403) and whereby the first and second inter-phase-gaps (403) are different;
    determining the slopes of the measured amplitude growth functions for the stimulation channel measured with the first and second inter-phase-gaps (403), and
    calculating an indicator based at least in part on the difference of the slopes of the amplitude growth functions indicative of the local neural survival for that stimulation channel; and
    repeating this process for each stimulation channel where an indicator shall be derived;
    selecting for the virtual channel matrix the stimulation channels with best local neural survival by optimizing a function based at least in part on the calculated indicators of the stimulation channels.

2. A method according to claim 1, wherein the first and second inter-phase-gaps (403) are at least 2 microseconds and/or the difference between the first and second inter-phase-gaps (403) is at least 8 microseconds.

3. A method according to claim 1, wherein the difference of the first and second inter-phase-gaps (403) is at least 7.9 microseconds and the inter-phase-gaps (403) are at least 2.1 microseconds.

4. A method according to claim 1, wherein the function further includes at least in part a component supporting uniform distribution of selected stimulation channels over the frequency range covered by the stimulation channels where an indicator shall be derived.

5. A method according to claim 1, wherein optimizing the function includes forming stimulation channel groups by grouping a pre-defined number of adjacent stimulation channels and selecting the stimulation channel with the largest calculated indicator in this group, reflecting the highest estimated local neuronal survival.

6. A method according to claim 1, wherein optimizing the function includes forming at least two stimulation channel groups by grouping a pre-defined number of adjacent stimulation channels and selection of one stimulation channel per group such that the variance of the calculated indicators from the selected stimulation channels for all groups is minimized.

7. A system for fitting a virtual channel matrix for mapping analysis channels to stimulation channels of an implantable hearing system with a cochlear implant for electrical stimulation of the cochlear, the system comprising:

at least one hardware implemented, and programmable processor adapted to perform the steps of:
selecting a stimulation channel and measuring the amplitude growth function for the selected stimulation channel in response to commanding the cochlear implant to apply electrical stimulation pulses for the stimulation channel, where each stimulation pulse comprises a negative (401, 401a, 401b) and positive (402) phase separated in time by a first inter-phase-gap (403);
measuring the amplitude growth function for the stimulation channel in response to commanding the cochlear implant to apply electrical stimulation pulses for the stimulation channel, where each stimulation pulse comprises a negative (401, 401a, 401b) and positive (402) phase separated in time by a second inter-phase-gap (403) and whereby the first and second inter-phase-gaps (403) are different;
determining the slopes of the measured amplitude growth functions for the stimulation channel measured with the first and second inter-phase-gaps (403), and
calculating an indicator based at least in part on the difference of the slopes of the amplitude growth functions indicative of the local neural survival for that stimulation channel; and
repeating this process for each stimulation channel where an indicator shall be derived;
selecting for the virtual channel matrix the stimulation channels with best local neural survival by optimizing a function based at least in part on the calculated indicators of the stimulation channels.

8. The system according to claim 7, wherein the hardware implemented, and programmable processor is further adapted to allow setting the first and second inter-phase-gaps (403).

9. The system according to claim 7, wherein the hardware implemented, and programmable processor is further adapted to store default inter-phase-gaps (403) and wherein the default inter-phase-gaps (403) for the first and second inter phase gaps (403) are at least 2.1 microseconds and the difference between the first and second inter-phase-gaps (403) is at least 7.9 microseconds.

10. The system according to claim 7, wherein the hardware implemented, and programmable processor is further adapted to apply an optimizing function that is further based at least in part on the uniform distribution of selected stimulation channels over the frequency range covered by the stimulation channels where an indicator shall be derived.

11. The system according to claim 7, wherein the hardware implemented, and programmable processor is further adapted to apply an optimizing function that includes forming stimulation channel groups by grouping a pre-defined number of adjacent stimulation channels and selecting the stimulation channel with the largest calculated indicator in this group, reflecting the highest estimated local neuronal survival.

12. The system according to claim 7, wherein the hardware implemented, and programmable processor is further adapted to apply an optimizing function that includes selecting the stimulation channels where the calculated indicator exceeds a pre-defined threshold.

13. The system according to claim 7, wherein the hardware implemented, and programmable processor is further adapted to apply an optimizing function that includes selecting the stimulation channels that minimize the variance of the calculated indicators.

14. The system according to claim 7, wherein the hardware implemented, and programmable processor is further adapted to apply an optimizing function that includes forming at least two stimulation channel groups by grouping a pre-defined number of adjacent stimulation channels and selection of one stimulation channel per group such that the variance of the calculated indicators from the selected stimulation channels for all groups is minimized.

15. A computer program product for fitting a hearing device of a patient, the computer program product comprising a computer usable medium having computer readable program code thereon, the computer readable program code comprising instructions for carrying out the method steps according to any of the claims 1 to 6

**Patentansprüche**

1. Verfahren zum Entwickeln einer virtuellen Kanalmatrix zum Zuordnen von Analysekanälen auf Stimulationskanäle für einen Cochlea-Implantat-Patienten, umfassend:

Auswählen eines Stimulationskanals und Messen der Amplitudenwachstumsfunktion für den ausgewählten Stimulationskanal in Reaktion auf Befehle an das Cochlea-Implantat elektrische Stimulationsimpulse für den Stimulationskanal anzulegen, wobei jeder Stimulationsimpuls eine negative (401, 401a, 401b) und positive (402) Phase umfasst, die durch einen ersten Zwischenphasenabstand (403) zeitlich getrennt sind;

Messen der Amplitudenwachstumsfunktion für den ausgewählten Stimulationskanal in Reaktion auf Befehle an das Cochlea-Implantat elektrische Stimulationsimpulse für den Stimulationskanal anzulegen, wobei jeder Stimulationsimpuls eine negative (401, 401a, 401b) und positive (402) Phase umfasst, die durch einen zweiten Zwischenphasenabstand (403) zeitlich getrennt sind, und wobei der erste und der zweite Zwischenphasenabstand (403) unterschiedlich sind;

Bestimmen der Steigungen der gemessenen Amplitudenwachstumsfunktionen für den Stimulationskanal, gemessen mit den ersten und zweiten Zwischenphasenabstand (403), und

Berechnen eines Indikators basierend zumindest teilweise auf der Differenz der Steigungen der Amplitudenwachstumsfunktionen, die das lokale neuronale Überleben für diesen Stimulationskanal anzeigen; und

Wiederholen dieses Prozesses für jeden Stimulationskanal, für welchen ein Indikator abgeleitet werden soll;

Auswählen der Stimulationskanäle für die virtuelle Kanalmatrix mit dem besten lokalen neuronalen Überleben durch Optimieren einer Funktion zumindest teilweise basierend auf den berechneten Indikatoren der Stimulationskanäle.

2. Verfahren nach Anspruch 1, wobei der erste und zweite Zwischenphasenabstand (403) zumindest 2 Mikrosekunden und/oder die Differenz zwischen dem ersten und zweiten Zwischenphasenabstand (403) zumindest 8 Mikrosekunden ist.

3. Verfahren nach Anspruch 1, wobei die Differenz der ersten und zweiten Zwischenphasenabstände (403) mindestens 7,9 Mikrosekunden beträgt und die Zwischenphasenabstände (403) mindestens 2,1 Mikrosekunden betragen.

4. Verfahren nach Anspruch 1, wobei die Funktion ferner zumindest teilweise eine Komponente umfasst, die eine gleichmäßige Verteilung ausgewählter Stimulationskanäle über den Frequenzbereich unterstützt, der von den Stimulationskanälen abgedeckt wird, für welche ein Indikator abgeleitet werden soll.

5. Verfahren nach Anspruch 1, wobei die Optimierungsfunktion das Bilden von Stimulationskanalgruppen durch Gruppieren einer vordefinierten Anzahl von benachbarten Stimulationskanälen und das Auswählen des Stimulationskanals mit dem größten berechneten Indikator in dieser Gruppe, welcher das höchste geschätzte lokale neuronale Überleben widerspiegelt.

6. Verfahren nach Anspruch 1, wobei die Optimierungsfunktion das Bilden von mindestens zwei Stimulationskanalgruppen durch Gruppieren einer vordefinierten Anzahl von benachbarten Stimulationskanälen und Auswählen eines Stimulationskanals pro Gruppe umfasst, so dass die Varianz der berechneten Indikatoren aus den ausgewählten Stimulationskanälen für alle Gruppen minimiert wird.

7. Ein System zum Anpassen einer virtuellen Kanalmatrix zum Abbilden von Analysekanälen auf Stimulationskanäle eines implantierbaren Hörsystems mit einem Cochlea-Implantat zur elektrischen Stimulation der Cochlea, wobei das System umfasst:

mindestens ein in Hardware realisierter und programmierbarer Prozessor, der angepasst ist, um die Schritte auszuführen:

Auswählen eines Stimulationskanals und Messen der Amplitudenwachstumsfunktion für den ausgewählten Stimulationskanal in Reaktion auf Befehle an das Cochlea-Implantat elektrische Stimulationsimpulse für den Stimulationskanal anzulegen, wobei jeder Stimulationsimpuls eine negative (401, 401a, 401b) und positive (402) Phase umfasst, die durch einen ersten Zwischenphasenabstand (403) zeitlich getrennt sind;

Messen der Amplitudenwachstumsfunktion für den ausgewählten Stimulationskanal in Reaktion auf Befehle an das Cochlea-Implantat elektrische Stimulationsimpulse für den Stimulationskanal anzulegen, wobei jeder Stimulationsimpuls eine negative (401, 401a, 401b) und positive (402) Phase umfasst, die durch einen zweiten Zwischenphasenabstand (403) zeitlich getrennt sind, und wobei der erste und der zweite Zwischenphasenabstand (403) unterschiedlich sind;

Bestimmen der Steigungen der gemessenen Amplitudenwachstumsfunktionen für den Stimulationskanal, gemessen mit den ersten und zweiten Zwischenphasenabstand (403), und

Berechnen eines Indikators basierend zumindest teilweise auf der Differenz der Steigungen der Amplitudenwachstumsfunktionen, die das lokale neuronale Überleben für diesen Stimulationskanal anzeigen; und

Wiederholen dieses Prozesses für jeden Stimulationskanal, für welchen ein Indikator abgeleitet werden soll;

Auswählen der Stimulationskanäle für die virtuelle Kanalmatrix mit dem besten lokalen neuronalen Überleben durch Optimieren einer Funktion zumindest teilweise basierend auf den berechneten Indikatoren der Stimulationskanäle.

8. System nach Anspruch 7, wobei der in Hardware realisierte und programmierbare Prozessor ferner angepasst ist, um die Einstellung der ersten und zweiten Zwischenphasenabstände (403) zu ermöglichen.

9. System nach Anspruch 7, wobei der in Hardware realisierte und programmierbare Prozessor ferner angepasst ist, die Standardzwischenphasenabstände (403) zu speichern, und wobei die standardmäßigen Zwischenphasenabstände (403) für den ersten und den zweiten Zwischenphasenabstand (403) mindestens 2,1 Mikrosekunden sind und die Differenz zwischen dem ersten und dem zweiten Zwischenphasenabstand (403) mindestens 7,9 Mikrosekunden beträgt.

10. System nach Anspruch 7, wobei der in Hardware realisierte und programmierbare Prozessor ferner angepasst ist, um eine Optimierungsfunktion anzuwenden, die ferner zumindest teilweise auf der gleichmäßigen Verteilung ausgewählter Stimulationskanäle über den Frequenzbereich basiert, der von den Stimulationskanälen abgedeckt wird, wofür ein Indikator abgeleitet werden soll.

11. System nach Anspruch 7, wobei der in Hardware realisierte und programmierbare Prozessor ferner angepasst ist, um eine Optimierungsfunktion anzuwenden, die das Bilden von Stimulationskanalgruppen durch Gruppieren einer vordefinierten Anzahl von benachbarten Stimulationskanälen und Auswählen des Stimulationskanals mit dem größten berechneten Indikator in dieser Gruppe umfasst, welcher das höchste geschätzte lokale neuronale Überleben widerspiegelt.

12. System nach Anspruch 7, wobei der in Hardware realisierte und programmierbare Prozessor ferner angepasst ist, eine Optimierungsfunktion anzuwenden, die das Auswählen der Stimulationskanäle beinhaltet, wobei der berechnete Indikator einen vordefinierten Schwellenwert überschreitet.

13. System nach Anspruch 7, wobei der in Hardware realisierte und programmierbare Prozessor ferner angepasst ist, eine Optimierungsfunktion anzuwenden, die das Auswählen der Stimulationskanäle beinhaltet, die die Varianz der berechneten Indikatoren minimieren.

14. System nach Anspruch 7, wobei der in Hardware realisierte und programmierbare Prozessor ferner angepasst ist, um eine Optimierungsfunktion anzuwenden, die das Bilden von mindestens zwei Stimulationskanalgruppen durch Gruppieren einer vordefinierten Anzahl von benachbarten Stimulationskanälen und Auswählen eines Stimulationskanals pro Gruppe umfasst, so dass die Varianz der berechneten Indikatoren aus den ausgewählten Stimulationskanälen für alle Gruppen minimiert wird.

15. Computerprogrammprodukt zum Anpassen eines Hörgeräts eines Patienten, wobei das Computerprogrammprodukt ein computerverwendbares Medium mit einem computerlesbaren Programmcode darauf umfasst, wobei der computerlesbare Programmcode Anweisungen zum Ausführen der Verfahrensschritte nach einem der Ansprüche 1 bis 6 umfasst.

**Revendications**

1. Procédé de développement d'une matrice de canaux virtuels pour attribuer des canaux d'analyse à des canaux de stimulation pour un patient implanté cochléaire, comprenant :

la sélection d'un canal de stimulation et la mesure de la fonction de croissance d'amplitude pour le canal de stimulation sélectionné en réponse à des commandes d'application à l'implant cochléaire d'impulsions de stimulation électrique pour le canal de stimulation, chaque impulsion de stimulation comprenant une phase négative (401, 401a, 401b) et une phase positive (402) qui sont séparées dans le temps par un premier espacement interphase (403);

mesurer la fonction de croissance d'amplitude pour le canal de stimulation sélectionné en réponse à des commandes d'application à l'implant cochléaire d'impulsions de stimulation électrique pour le canal de stimulation, dans lequel chaque impulsion de stimulation comprend une phase négative (401, 401a, 401b) et une phase positive (402) séparées dans le temps par un deuxième espacement interphase (403), et dans lequel le premier et le deuxième espacement interphase (403) sont différents;

déterminer les pentes des fonctions de croissance d'amplitude mesurées pour le canal de stimulation, mesurées avec le premier et le deuxième espacement interphase (403), et

calculer un indicateur basé au moins en partie sur la différence des pentes des fonctions de croissance d'amplitude indiquant la survie neuronale locale pour ce canal de stimulation; et

répéter ce processus pour chaque canal de stimulation pour lequel un indicateur doit être dérivé;

sélectionner les canaux de stimulation pour la matrice de canaux virtuels présentant la meilleure survie neuronale locale en optimisant une fonction basée au moins en partie sur les indicateurs calculés des canaux de stimulation.

2. Procédé selon la revendication 1, dans lequel le premier et le deuxième espacement interphase (403) sont d'au moins 2 microsecondes et/ou la différence entre le premier et le deuxième espacement interphase (403) est d'au moins 8 microsecondes.

3. Procédé selon la revendication 1, dans lequel la différence entre le premier et deuxième intervalles interphasiques (403) est d'au moins 7,9 microsecondes et les intervalles interphasiques (403) sont d'au moins 2,1 microsecondes.

4. Procédé selon la revendication 1, dans lequel la fonction comprend en outre au moins partiellement un composant qui supporte une distribution uniforme de canaux de stimulation sélectionnés sur la plage de fréquences couverte par les canaux de stimulation pour lesquels un indicateur doit être dérivé.

5. Procédé selon la revendication 1, dans lequel la fonction d'optimisation consiste à former des groupes de canaux de stimulation en regroupant un nombre prédéfini de canaux de stimulation adjacents et en sélectionnant le canal de stimulation ayant le plus grand indicateur calculé dans ce groupe, qui reflète la survie neuronale locale estimée la plus élevée.

6. Procédé selon la revendication 1, dans lequel la fonction d'optimisation comprend la formation d'au moins deux groupes de canaux de stimulation en regroupant un nombre prédéfini de canaux de stimulation adjacents et en sélectionnant un canal de stimulation par groupe, de sorte que la variance des indicateurs calculés à partir des canaux de stimulation sélectionnés soit minimisée pour tous les groupes.

7. Un système d'adaptation d'une matrice de canaux virtuels pour mapper des canaux d'analyse sur des canaux de stimulation d'un système auditif implantable comprenant un implant cochléaire pour la stimulation électrique de la cochlée, le système comprenant: au moins un processeur réalisé en matériel et programmable, adapté pour réaliser les étapes de:

sélectionner un canal de stimulation et mesurer la fonction de croissance d'amplitude pour le canal de stimulation sélectionné en réponse à des commandes adressées à l'implant cochléaire pour appliquer des impulsions de stimulation électrique au canal de stimulation, chaque impulsion de stimulation comprenant une phase négative (401, 401a, 401b) et une phase positive (402) séparées dans le temps par un premier déphasage (403);

mesurer la fonction de croissance d'amplitude pour le canal de stimulation sélectionné en réponse à des commandes d'application à l'implant cochléaire d'impulsions de stimulation électrique pour le canal de stimulation, dans lequel chaque impulsion de stimulation comprend une phase négative (401, 401a, 401b) et une phase positive (402) séparées dans le temps par un deuxième espacement interphase (403), et dans lequel le premier et le deuxième espacement interphase (403) sont différents;

déterminer les pentes des fonctions de croissance d'amplitude mesurées pour le canal de stimulation, mesurées avec le premier et le deuxième espacement interphase (403), et

calculer un indicateur basé au moins en partie sur la différence des pentes des fonctions de croissance d'amplitude indiquant la survie neuronale locale pour ce canal de stimulation; et

répéter ce processus pour chaque canal de stimulation pour lequel un indicateur doit être dérivé;

sélectionner les canaux de stimulation pour la matrice de canaux virtuels présentant la meilleure survie neuronale locale en optimisant une fonction basée au moins en partie sur les indicateurs calculés des canaux de stimulation.

8. Système selon la revendication 7, dans lequel le processeur réalisé en matériel et programmable est en outre adapté pour permettre le réglage des premier et deuxième espacements interphases (403).

9. Système selon la revendication 7, dans lequel le processeur réalisé en matériel et programmable est en outre adapté pour stocker les espacements interphase standard (403), et dans lequel les espacements interphase standard (403) pour le premier et le deuxième espacement interphase (403) sont d'au moins 2,1 microsecondes et la différence entre le premier et le deuxième espacement interphase (403) est d'au moins 7,9 microsecondes.

10. Système selon la revendication 7, dans lequel le processeur réalisé en matériel et programmable est en outre adapté pour appliquer une fonction d'optimisation qui est en outre basée au moins en partie sur la distribution uniforme de canaux de stimulation sélectionnés sur la plage de fréquences couverte par les canaux de stimulation, dont un indicateur doit être dérivé.

11. Système selon la revendication 7, dans lequel le processeur réalisé en matériel et programmable est en outre adapté pour appliquer une fonction d'optimisation comprenant la formation de groupes de canaux de stimulation en regroupant un nombre prédéfini de canaux de stimulation adjacents et en sélectionnant le canal de stimulation ayant l'indicateur calculé le plus grand dans ce groupe, qui reflète la survie neuronale locale estimée la plus élevée.

12. Système selon la revendication 7, dans lequel le processeur réalisé en matériel et programmable est en outre adapté pour appliquer une fonction d'optimisation impliquant la sélection des canaux de stimulation, l'indicateur calculé dépassant une valeur de seuil prédéfinie.

13. Système selon la revendication 7, dans lequel le processeur réalisé en matériel et programmable est en outre adapté pour appliquer une fonction d'optimisation impliquant la sélection des canaux de stimulation qui minimisent la variance des indicateurs calculés.

14. Système selon la revendication 7, dans lequel le processeur réalisé en matériel et programmable est en outre adapté pour appliquer une fonction d'optimisation comprenant la formation d'au moins deux groupes de canaux de stimulation en regroupant un nombre prédéfini de canaux de stimulation adjacents et en sélectionnant un canal de stimulation par groupe, de sorte que la variance des indicateurs calculés à partir des canaux de stimulation sélectionnés soit minimisée pour tous les groupes.

15. Produit de programme informatique pour adapter une prothèse auditive d'un patient, le produit de programme informatique comprenant un support utilisable par ordinateur avec un code de programme lisible par ordinateur sur celui-ci, dans lequel le code de programme lisible par ordinateur comprend des instructions pour exécuter les étapes de procédé selon l'une quelconque des revendications 1 à 6.

FIG. 1

Implant 205

Pulse Gen 204

Z

Y1  X1  YK  XS

Envelope detector 202

Stimulation Timing 203

*Fig. 2*

U1  UK

Preprocessor, Filter Bank, additional processing 201

S

*Fig. 3*

*Fig. 4*

*Fig. 5*

*Fig. 6*

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 9180295 B
- US 8532782 B
- US 20080021551 A
- US 20180311500 A
- US 6594525 B, Zierhofer

**Non-patent literature cited in the description**

- **KASTURI K. ; LOIZOU P.** Effect Of Filter Spacing On Melody Recognition: Acoustic And Electric Hearing. *Journal of Acoustic Society of America,* 2007, vol. 122 (2), EL 29-EL 34
- **GANTZ et al.** Intraoperative Measures of Electrically Evoked Auditory Nerve Compound Action Potentials. *American Journal of Otology,* 1994, vol. 15 (2), 137-144
- **BROWN et al.** Electrically Evoked Whole Nerve Action Potentials In Ineraid Cochlear Implant Users: Responses To Different Stimulating Electrode Configurations And Comparison To Psychophysical Responses. *Journal of Speech and Hearing Research,* June 1996, vol. 39, 453-467
- Messung des elektrisch evozierten Summenaktionspotentials des Hörnervs bei Patienten mit einem Cochlea-Implantat. **ZIMMERLING M.** PhD thesis. Universität Innsbruck, Institut für Angewandte Physik, 1999
- **SCHOESSER H ; ZIERHOFER C ; HOCHMAIR ES.** Measuring electrically evoked compound action potentials using triphasic pulses for the reduction of the residual stimulation artefact. *Conference on implantable auditory prostheses,* 2001
- Across-site patterns of electrically evoked compound action potential amplitude-growth functions in multichannel cochlear implant recipients and the effects of the interphase gap. **SCHVARTZ-LEYZAC KARA C et al.** HEARING RESEARCH. ELSEVIER SCIENCE PUBLISHERS, 10 August 2016, vol. 341, 50-65
- Effect of interphase gap and pulse duration on electrically evoked potentials is correlated with auditory nerve survival. **PRADO-GUITIERREZ et al.** HEARING RESEARCH. ELSEVIER SCIENCE PUBLISHERS, 01 May 2006, vol. 215, 47-55
- **DYAN RAMEKERS et al.** Auditory-Nerve Responses to Varied Inter-Phase Gap and Phase Duration of the Electric Pulse Stimulus as Predictors for Neuronal Degeneration. *JOURNAL OF THE ASSOCIATION FOR RESEARCH IN OTOLARYNGOLOGY,* 28 January 2014, vol. 15 (2), ISSN 1525-3961, 187-202